# FASCICULE DE BREVET EUROPEEN

(11) **EP 0 911 038 B1**
(45) Date de publication et mention de la délivrance du brevet: **12.07.2000**
(21) Numéro de dépôt: 98402480.2
(22) Date de dépôt: 06.10.1998
(51) Int. Cl.: A61K 47/06, A61K 31/70

(54) **Composition anhydre à base de lactulose**
Wasserfreies Mittel auf Lactulosebasis
Anhydrous composition comprising lactulose

(30) Priorité: 21.10.1997 FR 9713186
(43) Date de publication de la demande: 28.04.1999
(73) Titulaire: Salsarulo, Odette, 92100 Boulogne-Billancourt (FR); Salsarulo, Gérard, 92100 Boulogne-Billancourt (FR)
(72) Inventeur: Salsarulo, Odette, 92100 Boulogne-Billancourt (FR); Salsarulo, Gérard, 92100 Boulogne-Billancourt (FR)
(74) Mandataire: Gutmann, Ernest

(56) Documents cités:
- EP-A- 0 486 353
- WO-A-95/22976
- GB-A- 543 309

## Description

La lactulose est un laxatif reconnu, agissant par son pouvoir osmotique au niveau de l'intestin.

Galéniquement, le lactulose se présente habituellement :
- soit sous forme de solution à 50%
- soit sous forme pure en poudre, mais il est, dans ce dernier cas, ingéré par les patients avec de l'eau.

Sous ces présentations, les doses orales actives de lactulose sont élevées, allant de 10 à 30 grammes/jour (pour un adulte) en valeur poudre pure (ce qui oblige souvent à plusieurs prises dans la journée). L'administration par voie orale ne va pas sans problèmes relatifs à la tolérance du produit par l'organisme. Elle est susceptible d'entraîner des effets secondaires indésirables, tels que ballonnements ou flatulences.

L'utilisation de matières grasses pour enrober des principes actifs de médicament a déjà été suggérée dans certains documents de l'état de la technique.

Ainsi la demande de brevet GB 543309-A (EVANS) se rapporte à des compositions pharmaceutiques à base d'acide acétyl salicylique ou d'un principe actif analgésique analogue devant être retenu dans la bouche de l'utilisateur aussi longtemps que possible. A cet effet, le principe actif analgésique est mélangé à une matière grasse ou cireuse, par exemple du beurre de cacao ou une cire paraffinique « qui fond à la température du corps ou au-dessous de celle-ci » pour en masquer le goût sans incidence aucune sur son efficacité.

La demande de brevet britannique mentionne également qu'il a antérieurement été suggéré inhiber le gonflement ou la dissolution prématurés de la pectine sous forme de particules utilisées à titre de laxatif, au contact de l'eau ou des liquides aqueux, grâce à l'enrobage de ces particules par une ou plusieurs matières grasses.

Le brevet français n°9013619/2688706 décrit une composition ou forme galénique caractérisée par l'incorporation de lactulose anhydre dans un véhicule pharmaceutiquement acceptable par voie orale, également anhydre, ayant notamment un point de fusion entre 45°C et 60°C et consistant en un mélange d'hydrocarbures paraffiniques purifiées, remédiait déjà en grande partie aux inconvénients précités.

En effet outre que ces compositions pharmaceutiques à base de lactulose peuvent être ingérées sans eau, donc comme telles par les patients, les dosages quotidiens cliniquement utiles avaient pu être abaissés à de 5 à 10 grammes de lactulose par jour pour un adulte.

Selon la demande PCT WO 9522976-A (JOUVEINAL LABORATOIRES) le point de fusion du mélange d'hydrocarbures associé au lactulose dans la composition laxative du brevet français, supérieur à la température du corps humain, « laisse fortement douter de sa mise à disposition dans le tractus gastro-intestinal et, plus généralement, de l'acceptabilité gustative du produit ».

L'invention de la demande JOUVEINAL concerne des émulsions gélifiées associant de l'huile de paraffine liquide à une phase aqueuse, dans lesquelles le lactulose a été essentiellement utilisé en tant qu'édulcorant hypocalorique, en lieu et place du saccharose utilisé dans les émulsions gélifiées de l'état de la technique, en l'absence de tout agent tensio-actif.

L'invention a pour but de fournir une composition galénique encore plus efficace que celle du brevet français n°9013619/2688706. Elle vise à la réalisation de médicaments ayant des teneurs en lactulose permettant des administrations quotidiennes de lactulose encore plus faibles, tout en conservant une activité laxative équivalente.

La présente invention est un perfectionnement de la composition décrite dans le brevet français identifié ci-dessus. Elle résulte de la double découverte que :
- l'efficacité due à la pression osmotique mise en oeuvre lors de l'absorption du médicament du brevet précédent n'était pas affectée par l'utilisation pour le véhicule d'enrobage d'un mélange d'hydrocarbures paraffiniques, le plus souvent de distillats de carbures paraffiniques contenant de l'huile de paraffine, mais ayant une température de fusion plus basse, notamment de l'ordre de 37°C, au lieu de 45 à 60°C, et
- que le dosage cliniquement utile de lactulose administrable quotidiennement dans ces conditions pouvait encore être réduit davantage, entraînant une réduction encore plus forte des effets secondaires (ballonnements ou flatulences) du lactulose lorsqu'il est utilisé à hautes doses.

La composition selon l'invention qui contient du lactulose anhydre enrobé dans un véhicule également anhydre consistant en un mélange d'hydrocarbures paraffiniques purifiés pharmaceutiquement acceptables, est donc caractérisée en ce que le véhicule présente un point de fusion de l'ordre de 37°C ± 4°C, donc égale ou en tous les cas proche de la température corporelle.

Il est à remarquer que la température de fusion sus-indiquée, d'ordre de 37°C, correspond à un point de fusion capillaire mesurable avec une variabilité de ± 5% par la mise en oeuvre de la technique décrite dans la Pharmacopée Française, 10e édition, 1983, V.611.

Selon une caractéristique supplémentaire préférée de l'invention, le lactulose anhydre mis en oeuvre dans ces compositions est micronisé, les tailles de particules étant avantageusement comprises entre 75 et 150 µm. L'incorporation du lactulose anhydre ainsi micronisé au véhicule, en particulier l'enrobage de ses particules dans le véhicule, entraîne une plus grande dispersion du lactulose au sein de la composition et un accroissement supplémentaire de sa pression osmotique.

Il en résulte que les dosages cliniquement utiles de lactulose administrable à un adulte sous cette nouvelle forme peuvent être abaissés davantage encore, notamment être réduits à de 3,5 à 5 grammes par jour, en lieu et place des dosages quotidiens que requérait encore la mise en oeuvre des compositions du brevet précédent : 5 à 10 grammes de lactulose par jour pour un adulte.

Il va sans dire que la composition ainsi obtenue ne représente pas nécessairement la présentation pharmaceutique finale, telle qu'elle sera destinée aux patients. Celle-ci peut en outre contenir d'autres excipients, à condition que soit respecté le caractère anhydre du lactulose, base de sa pression osmotique, et dès lors que la composition finale comporte bien les doses utiles sus-indiquées de lactulose.

Comme dans le cas du précédent brevet, le véhicule à base d'hydrocarbures est avantageusement constitué de distillats anhydres de carbures paraffiniques, affinés à un niveau leur assurant une complète innocuité et, en tant que besoin, sélectionner pour que ce mélange ait une température de fusion de l'ordre de 37°C, par adjonction éventuelle et complémentaire d'huile de paraffine de qualité Pharmacopée. Le véhicule préféré est donc constitué par un mélange de distillats de carbures paraffiniques, contenant l'huile de paraffine, étant entendu que les proportions relatives de ces constituants sont à choisir par l'homme du métier en fonction de la température de fusion choisie, de l'ordre de 37°C. A titre d'exemple non limitatif on indiquera que la proportion d'huile de paraffine se trouvera, de ce fait, souvent être comprise dans une proportion variant de ¼ à ¾ du poids de la composition. Il va sans dire que l'huile de paraffine peut même être omise si le mélange de distillats de carbures paraffiniques présente déjà lui-même une température de fusion de l'ordre de 37°C.

Trois compositions satisfaisant aux conditions de l'invention sont présentées ci-après, à titre d'exemples, étant néanmoins entendu qu'ils ne sauraient en aucun cas être considérés comme ayant un caractère limitatif.

La proportion de l'enrobage (carbures paraffiniques et huile de paraffine) est d'environ 1 à 2 fois la quantité de lactulose. Ainsi la proportion de lactulose par rapport à l'ensemble de la composition est avantageusement de l'ordre de 30 à 45% en poids de la composition totale, et par exemple de l'ordre de 3,5 g de lactulose pour 6,5 g d'hydrocarbures.

L'utilisation de la composition selon l'invention, s'accompagne naturellement des avantages déjà signalés à propos de l'invention décrite dans le précédent brevet, mais à un degré encore plus bénéfique, puisqu'elle permet, à efficacité égale, une réduction supplémentaire de la posologie, en particulier grâce à la réduction supplémentaire des doses efficaces de lactulose, à de 3 à 5 grammes par jour, au lieu des 5 à 10 grammes par jour requis pour la composition du brevet antérieur.

Avantageusement, la composition est présentée sous une forme galénique telle qu'elle permet l'administration de doses efficaces quotidiennes d'environ 3,5 à 5 grammes de lactulose par jour, de préférence en une seule prise. A cette dose réduite la tolérance est fortement améliorée.

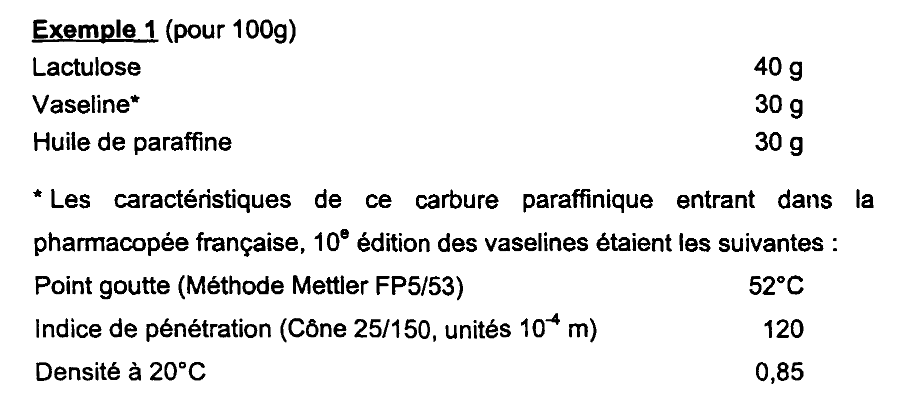

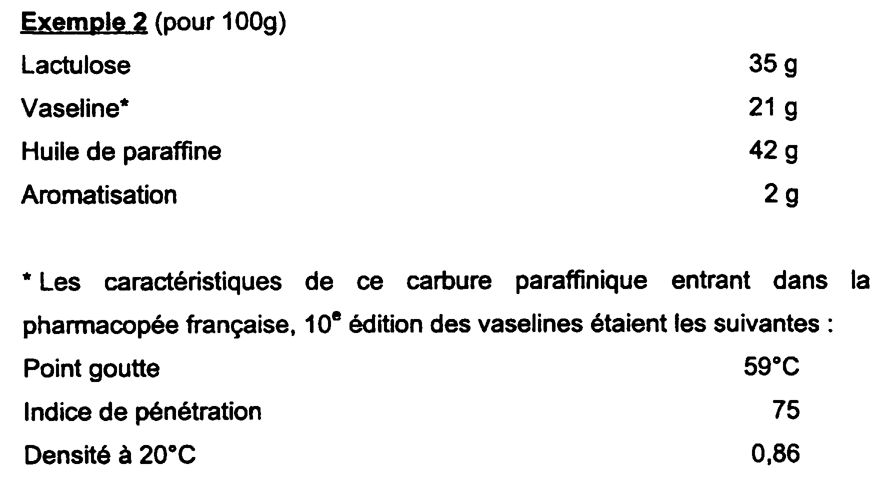

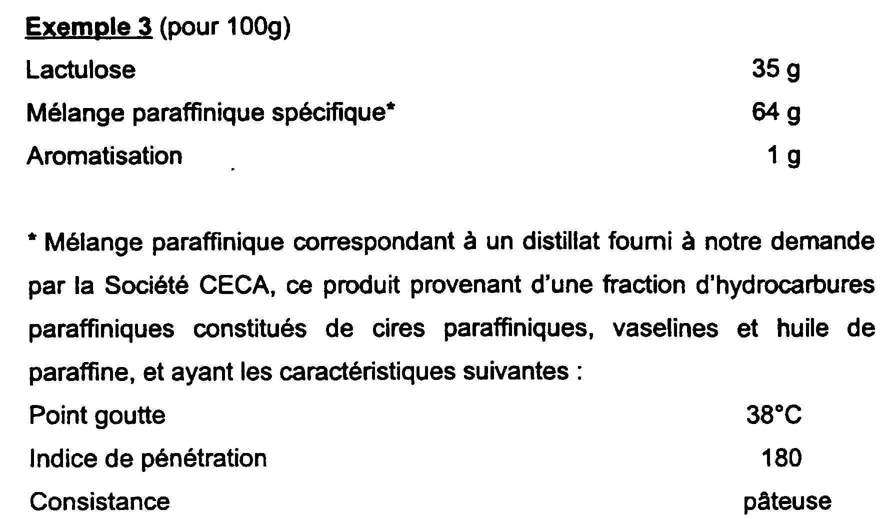

## Revendications

1. Composition à base de lactulose caractérisée par l'incorporation de lactulose anhydre dans un véhicule d'enrobage également anhydre consistant en un mélange d'hydrocarbures paraffiniques purifiés pharmaceutiquement acceptables, ce véhicule présentant un point de fusion de l'ordre de 37°C ± 4°C:

2. Composition selon la revendication 1, caractérisée par une micronisation du lactulose en particules de 75 à 150 micromètres (microns).

3. Composition selon la revendication 1 ou la revendication 2, caractérisée en ce que les particules sont dispersées et enrobées dans le véhicule anhydre.

4. Composition selon l'une quelconque des revendications 1 à 3, caractérisée en ce que la proportion de lactulose par rapport à l'ensemble de la composition est de l'ordre de 30 à 45% en poids de la composition totale.

5. Composition selon l'une quelconque des revendications 1 à 4, caractérisée par une teneur en hydrocarbures paraffiniques égale à 1 à 2 fois la teneur en lactulose.

6. Composition selon la revendication 5, caractérisée en ce qu'elle contient de l'ordre de 3,5 grammes de lactulose pour environ 6,5 grammes d'hydrocarbures paraffiniques pharmaceutiquement acceptables.

7. Composition selon l'une quelconque des revendications 1 à 4, caractérisée en ce qu'elle permet l'administration d'une dose efficace de lactulose à raison de 3,5 à 5 grammes par jour, chez l'adulte.

8. Médicament laxatif anhydre, ingérable sans eau, pour l'administration orale, contenant une composition selon l'une quelconque des revendications 1 à 6.

9. Médicament selon la revendication 8, caractérisé en ce qu'il est présenté sous une forme galénique permettant l'administration d'une dose efficace à raison de 3 à 5g de lactulose par jour.

10. Médicament selon la revendication 9, caractérisé en qu'il est sous une forme permettant l'administration à la dose susdite, en une seule prise quotidienne.

11. Procédé de production d'un médicament anhydre laxatif, à base de lactulose, caractérisé par l'association du lactulose sous une forme ingérable sans eau par les patients, avec un mélange d'hydrocarbures paraffiniques purifiés pharmaceutiquement acceptables, notamment tel que défini dans l'une quelconque des revendications 1 à 7.

## Patentansprüche

1. Zusammensetzung auf Basis von Lactulose, dadurch gekennzeichnet, dass wasserfreie Lactulose in ein ebenfalls wasserfreies Umhüllungsträgermittel eingebracht wird, das aus einer Mischung aus paraffinischen gereinigten pharmazeutisch zulässigen Kohlenwasserstoffen besteht, wobei dieses Trägermittel einen Schmelzpunkt in der Größenordnung von 37°C ± 4°C aufweist.

2. Zusammensetzung gemäß Anspruch 1,
dadurch **gekennzeichnet**, dass die Lactulose in Teilchen von 75 bis 150 µm zerkleinert ist.

3. Zusammensetzung gemäß Anspruch 1 oder 2,
dadurch **gekennzeichnet**, dass die Teilchen in dem wasserfreien Trägermittel dispergiert und eingehüllt sind.

4. Zusammensetzung gemäß einem der Ansprüche 1 bis 3,
dadurch **gekennzeichnet**, dass der Mengenanteil von Lactulose, bezogen auf die gesamte Zusammensetzung, in der Größenordnung von 30 bis 45 Gew.% der gesamten Zusammensetzung liegt.

5. Zusammensetzung gemäß einem der Ansprüche 1 bis 4,
**gekennzeichnet** durch einen Gehalt an paraffinischen Kohlenwasserstoffen von gleich dem 1 - bis 2-fachen Gehalt an Lactulose.

6. Zusammensetzung gemäß Anspruch 5,
dadurch **gekennzeichnet**, dass sie ungefähr 3,5 g Lactulose auf ca. 6,5 g paraffiniache pharmazeutisch zulässige Kohlenwasserstoffe enthält.

7. , Zusammensetzung gemäß einem der Ansprüche 1 bis 4,
dadurch **gekennzeichnet**, dass sie mit einer wirksamen Dosis an Lactulose in einer Menge von 3,5 bis 5 g/Tag bei Erwachsenen verabreicht wird.

8. Wasserfreies Abführmittel-Medikament, das ohne Wasser einnehmbar ist, zur oralen Verabreichung, enthaltend eine Zusammensetzung gemäss einem der Ansprüche 1 bis 6.

9. Medikament gemäss Anspruch 8,
dadurch **gekennzeichnet**, dass es in galenischer Form vorliegt, die mit einer wirksamen Dosismenge von 3 bis 5 g Lactulose pro Tag verabreicht wird.

10. Medikament gemäss Anspruch 9,
dadurch **gekennzeichnet**, dass es in einer Form vorliegt, die mit der oben genannten Dosis in einer einzigen täglichen Einnahmemenge verabreicht wird.

11. Verfahren zur Herstellung eines wasserfreien Abführmittel-Medikaments auf Basis von Lactulose,
**gekennzeichnet** durch die in einer von den Patienten ohne Wasser einnehmbaren Form vorliegende Abmischung von Lactulose mit einer Mischung aus paraffinischen gereinigten pharmazeutisch zulässigen Kohlenwasserstoffen, welche in einem der Ansprüche 1 bis 7 definiert ist.

## Claims

1. Lactulose-based composition, characterized by the incorporation of anhydrous lactulose into a coating vehicle, which is also anhydrous, consisting of a mixture of pharmaceutically acceptable purified paraffinic hydrocarbons, this vehicle having a melting point of about 37°C ± 4°C.

2. Composition according to Claim 1, characterized by a micronization of the lactulose into particles of 75 to 150 micrometers (microns).

3. Composition according to Claim 1 or Claim 2, characterized in that the particles are dispersed and coated in the anhydrous vehicle.

4. Composition according to any one of Claims 1 to 3, characterized in that the proportion of lactulose relative to the entire composition is from about 30 to 45% by weight of the total composition.

5. Composition according to any one of Claims 1 to 4, characterized by a paraffinic hydrocarbon content equal to 1 to 2 times the lactulose content.

6. Composition according to Claim 5, characterized in that it contains about 3.5 grams of lactulose for about 6.5 grams of pharmaceutically acceptable paraffinic hydrocarbons.

7. Composition according to any one of Claims 1 to 4, characterized in that it allows the administration of an effective dose of lactulose in a proportion of 3.5 to 5 grams per day to adults.

8. Anhydrous laxative medicinal product which can be ingested without water, for oral administration, containing a composition according to any one of Claims 1 to 6.

9. Medicinal product according to Claim 8, characterized in that it is in a pharmaceutical form which allows administration of an effective dose in a proportion of 3 to 5 g of lactulose per day.

10. Medicinal product according to Claim 9, characterized in that it is in a form which allows administration at the abovementioned dose in a single daily dosage intake.

11. Process for producing an anhydrous, laxative, lactulose-based medicinal product, characterized by the combination of lactulose, in a form which patients can ingest without water, with a mixture of pharmaceutically acceptable purified paraffinic hydrocarbons, in particular as defined in any one of Claims 1 to 7.
